# EUROPEAN PATENT APPLICATION

(11) **EP 1 252 886 A1**
(43) Date of publication of application: **30.10.2002**
(21) Application number: 01201554.1
(22) Date of filing: 27.04.2001
(51) Int. Cl.: A61K 9/16

(54) **Controlled release pharmaceutical pellet compositions**

(71) Applicant: Universiteit Gent, 9000 Gent (BE)
(72) Inventor: Remon, Jean Paul, 9090 Melle (BE); Debunne, Ann, 9050 Gentbrugge (BE)
(74) Representative: Bird, Ariane

(57) **Abstract**

The invention provides a controlled release pharmaceutical pellet composition comprising a non-steroidal anti-inflammatory drug, the said composition providing a release of at least 75% of the non-steroidal anti-inflammatory drug within 45 minutes in phosphate buffer pH 6.8, further comprising a microcrystalline cellulose and a swellable polymer in respective amounts such that the weight ratio of the swellable polymer to the microcrystalline cellulose is from 0.3 :100 to 30 :100.

## Description

The present invention is in the field of drug delivery systems and controlled release technology. In particular, the invention is in the field of controlled release pharmaceutical compositions and more specifically in relation with non-steroidal anti-inflammatory drugs. In addition to controlled release pharmaceutical pellet compositions, this invention relates to solid shaped articles containing them and to a process for manufacturing them.

### BACKGROUND OF THE INVENTION

Non-steroidal anti-inflammatory drugs are used in humans and animals for the treatment of inflammatory and rheumatic disorders such as dysplasia of the hip, chronic arthritis, spondylitis and the like. As is well known to those skilled in the art, one of the most commonly signaled side effects of non-steroidal anti-inflammatory drugs is irritation of the gastrointestinal tract. This side effect can be attributed to local irritation due to the penetration of the drug in the gastric mucosal cells, but it can also be caused by enterohepatic recirculation, which extends the contact period of the non-steroidal anti-inflammatory drug with the mucosa, or by the inhibition of prostaglandin synthesis due to the inhibition of the cyclooxygenase enzyme.

In order to solve this problem of local irritation caused by non-steroidal anti-inflammatory drugs, it is desirable to provide a suitable drug delivery system, for instance a system by which the said drug will not, or hardly, come in contact with the gastric mucosa and/or by which high local drug concentrations are avoided and/or by which the gastric emptying time will be less variable than with the existing formulations.

Some general considerations relating to drug formulation are now provided herein in order to understand the applicable constraints to which a solution to the above problem is faced and at the same time the kind of formulations to which the present invention may be applied.

Tablets and capsules are generally unsuitable for administering high doses of biologically active ingredients such as non-steroidal anti-inflammatory drugs since individual large dosage forms are difficult to swallow or necessitate the administration of several tablets or capsules at a time, leading to impaired patient compliance. Chewable tablets are usually not suitable with young children and older people and are furthermore unsuitable for the incorporation of controlled-release coated pellets which could get crushed upon chewing.

Oral liquid suspensions of pharmaceutical and veterinary ingredients are designed for those experiencing difficulty in swallowing solid medication but are not suitable for the incorporation of controlled-release particles into aqueous vehicles, since this often results in premature release of the active ingredient into the suspending media during storage. Efforts made to formulate sustained-release suspensions include using ion-exchange resins in order to bind charged molecules but this has limitations including low drug-loading capability and applicability to ionic drugs only.

The formulation of a solid oral dosage form, whether tablet or capsule, which disintegrates rapidly in water to form an instantaneous homogenous suspension of adequate viscosity to be swallowed could circumvent the problems of administering large dosages without premature release from controlled-release particles while providing a ready measured dose. The key to the development of such a dosage form is a rapidly disintegrating tablet which disperses to form a viscous suspension. A delay in the development of a viscous gel is essential for achieving disintegration of the tablet. On the other hand, a rapidly increasing viscosity is necessary to provide adequate suspension properties.

The ideal solid oral dosage form should contain a swellable material which is able to increase viscosity on contact with water, an active ingredient for controlled or sustained release delivery and a filler conferring compactibility and the capability to disintegrate quickly. The inclusion of a viscosity increasing agent as a fine powder in the tablet matrix without any processing would interfere with disintegration and result in the formation of a voluminous hydrophilic mass which is impossible to disperse. Thus, it is necessary to incorporate such an agent into the tablet as granules or spheres so that the disintegration process occurs before the viscosity increase.

Hard gelatin capsules are also known as a pharmaceutical dosage form. Their sizes have been standard since the start of industrial manufacture, ranging from 5 (corresponding to a volume of 0.13 ml) up to 000 (volume of 1.36 ml). Thus, when a large amount of ingredient is required for each dosage unit, depending on the bulk density of the formulation, it may be necessary to use large size capsules which are too large to swallow or, even worse, a size 000 capsule may be too small to receive the said amount. Pellets and coated pellets have often been filled into hard gelatin capsules to be used as conventional or controlled release dosage forms, however it is rather difficult to manufacture sustained-release formulations while using a hard gelatin capsule as the dosage form and such attempts have found relatively limited use despite efforts to improve the engineering of such formulations. This is why tablets are generally recognized as the most popular pharmaceutical oral dosage form participating in the comfort of the patient. This is especially true of sustained-release tablets which are designed to release the drug slowly after ingestion. In this case, patient compliance is improved since the daily number of tablets and the frequency with which the patient has to take these tablets to obtain the desired effect are considerably reduced. With sustained-release tablets, drug activity can be extended to take effect throughout the night, so that the patient need not be awakened until morning, thus resulting in time saving for nurses in hospitals.

The concept of tabletting coated active ingredient particles is therefore of interest. Attempts have been made to produce tablets comprising microcapsules because of the advantages resulting from the microencapsulated substance being protected from external influences and vice-versa, e.g. increased stability, reduced chances of irritations or undesirable reactions with other components in a mixture, ability to mask unpleasant tastes and smells, etc. However, compaction of coated beads (i.e. pellets) for making tablets encounters difficulties or problems. If the beads have been coated by a rate-controlling polymeric coating to sustain active ingredient delivery, cracking of the coating will cause the delivery system to change the rate of active ingredient delivery or controlledly release the dose. In "Design of an oral sustained release drug delivery system comprising polymer coated pellets compacted into tablet", Dyer et al. describe the production of a tablet formed from pellets containing active ingredient. The pellets are coated with a polymer which forms a controlled release membrane and, on oral administration of the tablets, intact polymer coated pellets are released in which the controlled release membranes are preserved. The pellets are formed by extrusion-spheronization of a mixture of 80% ibuprofen and 20% microcrystalline cellulose. The spheres were subsequently coated with a film including triethyl citrate plasticizer. The pellets were then introduced into a tabletting machine with a mixture of excipients in a total amount adequate to fill the void volume. Release profiles of active ingredient from the tablets indicate that the film coat is damaged during the tabletting procedure, resulting in increase in release rate. Scanning electron microscopy showed that damage was incurred by pellets particularly at the tablet surface. Preventing cracking of the coating is therefore of utmost importance. Large amounts of carriers have been found necessary in most cases in order to overcome the tendency of microcapsules or coated beads to brittleness by preventing their rupture on compression, thus resulting again in unacceptably large tablets.

As is well known in the art, beads (or pellets) are quite distinguishable from granules. Pelletization is an agglomeration process that converts fine powders or granules into small, free-flowing, spherical or semi-spherical units. As opposed to the process of granulation, the production of beads results in a narrow size-range distribution. The more spherical nature of beads, as compared to granules, provides better flow and reduces segregation due to shape differences. Also, the surface morphology of beads is optimal for applying a functional coating.

Compaction of controlled release tablets containing coated pellets involves the following critical aspects. When such a dosage form is developed, the coated pellets must withstand the process of compaction without being damaged in order to prevent any undesirable effects on the active ingredient release properties. The type and amount of coating agent, the size of the sub-unit, the selection of external additives and the rate and magnitude of the applied pressure must be carefully considered. The process of bead compaction involves the application of stress to polymer-coated spherical cores. The desirable mechanical properties of coated beads to be compacted into a tablet together with excipients should be such that they are strong, not brittle and have low elastic resilience. Investigation of the mechanical properties of both uncoated and coated beads has demonstrated that the presence of a film coat applied by means of an aqueous polymeric dispersion of polymethacrylates influenced the crushing strength and the elastic properties of beads: increasing the polymer loading results in increasing the crushing strength of beads, whilst simultaneously enhancing bead resilience (characterized by a reduction in the elastic modulus).

Significant changes were observed between the compaction properties of the powder and pellet forms of the same formulations: powder formulations deformed plastically and produced stronger compacts, whereas their pellet corresponding forms exhibited elastic deformation and brittle fragmentation, which resulted in compacts of lower tensile strength. It was also observed that the active ingredient release rate from spheres coated with acrylate polymers increased with an initial increase in the applied pressure - this being attributed to the cracks in the coat that formed during compaction - but that further increase in pressure again retarded the release profile, possibly due to closer inter-particulate contacts within the tablet which partly compensated for the leaks of the pellet coats.

The selection of external additives is also of importance in the design of tablets since these additives are expected to prevent the occurrence of film cracking in the coated sub-units. Their compatibility with the active ingredient-loaded pellets, in terms of particle size, is also very critical since a non-uniform size distribution can cause segregation, resulting in tabletting problems such as weight variation, poor content uniformity, etc.

As previously mentioned, conventional highly compactible fillers like microcrystalline cellulose can be mixed with active ingredient-loaded beads and compressed into tablets. It is well known that beads made from microcrystalline cellulose are, by virtue of the inherent bonding capacity of this material, very hard and not easily deformed or broken. However due to particle size differences with active ingredient-loaded beads, segregation occurs and results in weight variation and content uniformity problems. Microcrystalline cellulose granules produced by dry or wet granulation techniques and having similar size as the active ingredient-loaded beads are able to minimize segregation due to size differences and subsequent problems. However this advantage is obtained to the detriment of compactibility.

Thus a first problem to be addressed by the present invention is the design of a drug delivery system which is suitable for suppressing or at least extensively decreasing local irritation caused by non-steroidal anti-inflammatory drugs. Another problem is the design of a drug delivery system based on pellets and providing a controlled release of a non-steroidal anti-inflammatory drug, preferably a release of at least 75% of the said drug within 45 minutes in phosphate buffer pH 6.8.

### SUMMARY OF THE INVENTION

The present invention is based on the unexpected observation that the above-identified problems may be solved by incorporating a non-steroidal anti-inflammatory drug into a pellet composition further comprising a selected combination of a microcrystalline cellulose and at least one suitable adjuvant, i.e. more precisely comprising suitable respective amounts of a microcrystalline cellulose and a swellable polymer and/or a drug dissolution enhancing amount of a cyclodextrin derivative.

More specifically, the invention first provides a controlled release pharmaceutical pellet composition comprising a non-steroidal anti-inflammatory drug, the said composition providing a release of at least 75% of the non-steroidal anti-inflammatory drug within 45 minutes in phosphate buffer pH 6.8, further comprising a microcrystalline cellulose and a swellable polymer in respective amounts such that the weight ratio of the swellable polymer to the microcrystalline cellulose is from about 0.3 :100 to about 30 :100, preferably from about 4 :100 to about 20 :100 and more preferably from about 5 :100 to about 15 :100. Preferably, the swellable polymer is a carboxyalkylcellulose metal salt such as sodium or calcium carboxymethylcellulose.

The present invention further provides a controlled release pharmaceutical pellet composition such as above and further comprising a drug dissolution enhancing agent such as a cyclodextrin or a cyclodextrin derivative, the weight ratio of the drug dissolution enhancing agent to the microcrystalline cellulose being preferably up to 50 :100.

The present invention further provides a controlled release pharmaceutical pellet composition comprising a non-steroidal anti-inflammatory drug, the said composition providing a release of at least 75% of the non-steroidal anti-inflammatory drug within 45 minutes in phosphate buffer pH 6.8, further comprising a microcrystalline cellulose and a drug dissolution enhancing amount of a cyclodextrin derivative, the cyclodextrin derivative preferably being hydroxypropyl-β-cyclodextrin. In this embodiment of the invention, the cyclodextrin derivative and the microcrystalline cellulose preferably are in respective amounts such that the weight ratio of the cyclodextrin derivative to the microcrystalline cellulose is at least about 15 :100.

The present invention further provides controlled release pharmaceutical pellet compositions wherein the non-steroidal anti-inflammatory drug constitutes from about 0.5 to 60% by weight of the composition.

The present invention further provides solid shaped articles, such as for instance tablets optionally comprising a coating, including a controlled release pharmaceutical pellet composition such as previously defined.

The present invention also provides the use of a mixture comprising a microcrystalline cellulose and (a) a swellable polymer in respective amounts such that the weight ratio of the swellable polymer to the microcrystalline cellulose is from 0.3 :100 to 30 :100 and/or (b) a drug dissolution enhancing amount of a cyclodextrin derivative, for the manufacture of a medicament based on a non-steroidal anti-inflammatory drug and providing release of at least 75% of the said drug within 45 minutes in phosphate buffer pH 6.8. The said medicament is preferably for the treatment of inflammatory diseases and rheumatic disorders such as dysplasia of the hip, chronic arthritis, spondylitis and the like.

The present invention further provides a method for reducing side effects of a non-steroidal anti-inflammatory drug, comprising providing a dosage form including a controlled release pharmaceutical pellet composition such as above and administering said dosage form to a human or mammal patient orally.

### BRIEF DESCRIPTION OF THE DRAWINGS

Figure 1 represents the release, as a function of time, of piroxicam from a comparative pellet composition and from a pellet composition according to the invention.
Figure 2 represents the release, as a function of time, of piroxicam from a comparative pellet composition and from various pellet compositions including sodium carboxymethylcellulose according to the invention.
Figure 3 represents the release, as a function of time, of piroxicam from a comparative pellet composition and from yet other pellet compositions including sodium carboxymethylcellulose and/or hydroxypropyl-β-cyclodextrin according to the invention.

### DETAILED DESCRIPTION OF THE INVENTION

For a detailed understanding of the present invention, the nature of the ingredients of the pharmaceutical pellet compositions and of their manufacturing methods are explained as follows.

Microcrystalline cellulose, in particular a pharmaceutical grade thereof, is well known in the art and is available from a variety of commercial sources, e.g. Avicel ® PH 101 (FMC), Emococel ® (Mendell), Vivocel ® (JRS) and the like.

Suitable swellable polymers such as the sodium carboxymethylcellulose commercially available under the tradename Nymcel ® or coprocessed with microcrystalline cellulose, e.g. Avicel ® RC 581 and Avicel ® CL 611 (FMC), are also well known in the art. The amount of such swellable polymer in the pharmaceutical pellet compositions of the invention is critical with respect to the amount of microcrystalline cellulose in order to achieve the desired release rate of at least 75% of the non-steroidal anti-inflammatory drug within 45 minutes in phosphate buffer pH 6.8, i.e. in order to reduce the well known side effects, namely local irritation of the gastrointestinal tract, of a non-steroidal anti-inflammatory drug.

Cyclodextrins and cyclodextrin derivatives, in particular their pharmaceutical grades as used in the present invention, are well known in the art and available from a variety of commercial sources. They may be collectively referred as starch cyclic degradation products containing 6 to 8 glucose residues, or alternatively as cyclic oligosaccharides composed of L-glucose molecules linked by α or β osidic bonds having a toric form.

Non-steroidal anti-inflammatory drugs considered within the framework of the present invention constitute a well-known class of therapeutic agents including sub-groups such as aminoarylcarboxylic acid derivatives, arylcarboxylic acid derivatives, pyrazoles, pyrazolones, thiazine carboxamides, and pharmaceutically acceptable salts and enantiomers thereof. Specific examples of such drugs include aceclofenac, acemetacin, alclofenac, amfenac, bromfenac, cinmetacin, clopirac, diclofenac sodium, etodolac, felbinac, fenclozic acid, fentiazac, glucametacin, ibufenac, indomethacin, isofezolac, isozepac, lonazolac, metiazinic acid, mofezolac, oxametacine, pirazolac, proglumetacin, sulindac, tiaramide, tolmetin, tropesin, zomepirac, bumadizon, butibufen, fenbufen, xenbucin, clidanac, ketorolac, tinoridine, alminoprofen, benoxaprofen, bermoprofen, bucloxic acid, carprofen, fenoprofen, flunoxaprofen, flurbiprofen, ibuprofen, indoprofen, ketoprofen, loxoprofen, naproxen, oxaprozin, piketoprofen, pirprofen, pranoprofen, protizinic acid, suprofen, tiaprofenic acid, ximoprofen, zaltoprofen, difenamizole, epirizole, apazone, benzpiperylon, feprazone, mofebutazone, oxyphenbutazone, phenylbutazone, pipebuzone, propylphenazone, ramifenazone, suxibuzone, thiazolinobutazone, aspirin, benorylate, bromosaligenin, calcium or lysine acetylsalicylate, diflunisal, etersalate, fendosal, gentisic acid, glycol salicylate, imidazole salicylate, mesalamine, morpholine salicylate, 1-naphtyl salicylate, olsalazine, parsalmide, phenyl acetylsalicylate, salacetamide, salsalate, sulfasalazine, ampiroxicam, droxicam, isoxicam, lornoxicam, piroxicam, tenoxicam, meloxicam and the like.

The amount of non-steroidal anti-inflammatory drug used in the pharmaceutical pellet compositions of the invention is not critical and may typically vary from about 0.5 to 60% by weight of the said composition.

The pharmaceutical pellet compositions of the invention may further optionally comprise one or more fillers. The nature and amount of such fillers are not critical to the present invention. They include for instance binding agents such as starch, gelatin, glucose, alginic acid, sodium and calcium alginates, water-soluble acrylic polymer, polyvinylpyrrolidone, ethylcellulose, hydroxypropylmethylcellulose and the like, glidants such as fumed (colloidal) silica (such as available under the tradename Aerosil ®), lubricants such as magnesium stearate, talc, sodium and magnesium lauryl sulfates, water-insoluble diluents such as dicalcium phosphate and water-soluble diluents such as lactose, sorbitol and the like.

In view of manufacturing the pharmaceutical pellet compositions of the invention, it is preferred to make use of an extrusion-spheronization process which may comprise the steps of:
(a) granulating a mixture of a non-steroidal anti-inflammatory drug, a microcrystalline cellulose, a swellable polymer and/or a cyclodextrin derivative, and optionally conventional additives (such as fillers) in the presence of a granulating fluid, and
(b) extruding the wet mass from step (a) by means of an extruder and then spheronizing the resulting extrudate by means of a spheronizer. Spheronization was first disclosed in U.S. Pat. No. 3,277,520 and equipment design change has been minimal since then. The spheronizer consists basically of a grooved horizontal plate rotating at high speed within a stationary vertical cylinder fitted with a door to allow release of the pellets. Although extrusion is usually regarded as a continuous process, spheronization equipment design limits the extrusion-spheronization process to a batch process or multiple batch process. There are five unit operations involved in the extrusion-spheronization process:
   blending or mixing the ingredient powders, wet granulation, extrusion, spheronization and drying. The moistened pre-compacted mass is extruded into
   strands, which are then rounded into pellets in a spheronization machine, dried and
   subjected to further processing. The granulating fluid may be water or an aqueous solution containing a lower alcohol, such as ethanol or propanol. The amount of granulating fluid used influences the mechanical properties (porosity, density, friability and compactibility) of the pellets produced. The amount of granulating fluid used depends on the composition of the powder mixture used in step (a) and is generally such as to provide a final solids concentration, of about 20 to 80% by weight. The granulating fluid content and the composition of the powdery mixture granulated in step (a) must be carefully selected in order that a suitable plastic deformability (extrudability) is obtained. The particle size distribution of the pellets obtained is also primarily determined by the extrudate density and granulating fluid content. However, individual process parameters such as the pressure at which the granulate of step (a) is extruded, the rotation speed of the spheronizer, the aperture size of the extruder screen of the spheronizer, the size and texture of the friction plate of the spheronizer and the spheronizer residence time are not critical to the present invention. The drying unit operation is usually effected by fluid bed, tray or freeze-drying and carried out at temperatures below the freezing point of the product, thus producing highly porous and compactible pellets.

The term "solid shaped article" as used herein means any article being in a hard solid state at temperatures not exceeding about 60°C and having a definite geometrical shape, such as for instance ordinary tablets, effervescent tablets, pills, lozenges and other compressed dosage forms. The solid shaped articles of the present invention preferably contains pellets (beads) having a diameter ranging from about 0.5 to about 2.0 mm and most preferably from 0.8 to 1.2 mm. The solid shaped articles of the present invention may further optionally contain additives typically used in the formulation of such articles, for instance flavoring agents (such as anethole, benzaldehyde, vanillin, ethyl vanillin, ethyl acetate, methyl salicylate and the like), lubricants (such as magnesium stearate), sweeteners (such as sucrose, mannitol, aspartame, saccharin and its salts), colorants and/or buffering agents.

Optionally, a coating material may be applied, preferably by means of the film-coating process, to the pellets for further controlling the release properties of the active ingredient or for taste masking or for imparting resistance to gastric fluid. Film coating of a tablet involves the deposition, usually by spraying, of a thin film of polymer surrounding the tablet core. The coating solution contains a polymer in a suitable liquid solvent and optionally mixed together with other ingredients such as plasticizers, pigments and/or colorants. After spraying, the drying conditions permit to remove substantially all of the solvent.

The particular coating material used is not critical to the present invention, and depends upon the purpose of the coating material, e.g. the release profile, the ability to stay intact and/or to withstand the mechanical stress of compaction without cracking and so on. Examples of coating polymers useful for controlling the release properties of the active ingredient and/or taste masking include derivatives of cellulose such as methylcellulose, hydroxypropylmethylcellulose and ethylcellulose, such as those marketed under the tradenames Surelease® and Aquacoat ®, polyvinylpyrrolidone and aminoalkyl methylacrylate copolymers. Examples of coating polymers useful for imparting resistance to gastric fluid include shellac, cellulose acetate phthalate (Aquateric®), cellulose acetate trimelliate, hydroxypropylmethylcellulose phthalate, polyvinyl acetate phthalate (Coateric®), hydroxypropyl methylcellulose acetate succinate, carboxymethylethylcellulose, styrene/acrylic acid copolymers, methacrylic acid copolymers, maleic anhydride copolymers and the like. Examples of plasticizers which may be mixed together with the coating polymer include, without limitation, polyethyleneglycol, glycerol, phtalate esters, triethylcitrate, etc.

The thickness of the coating layer used is not critical to the present invention. It depends upon the desired release profile of the active ingredient and typically is in the nanometer to micron ranges. Alternatively, the above-listed polymers and optionally plasticizers can be incorporated into a matrix system together with the active ingredient-loaded pellets to sustain its action, e.g. during dry powder mixing prior to granulation, or in the granulation solution prior to extrusion-spheronization, or within the other techniques conventionally used to produce pellets. In such a case, the amount of polymers and optionally plasticizers is not critical to the present invention, as long as they do not interfere with the desired release profile of the active ingredient.

Preferably, the applied coating is a film of polymeric gastroresistant and enterosoluble material, in order to allow activation of the pharmaceutical composition only after the solid shaped article has reached the duodenal-intestinal tract, i.e. more preferably, in order to release the active ingredient in the last part of the duodenal-intestinal tract. Cellulose acetophtalate, cellulose acetopropionate, cellulose trimellitate, acrylic and methacrylic polymers and copolymers having different molecular weight and solubility depending on pH values may be used for this purpose.

The following examples are provided solely for the purpose of illustrating various embodiments of the invention, and without any intention of limiting the scope thereof.

### EXAMPLE 1 - production of a pellet composition

Pellets are produced by means of extrusion-spheronization comprising the steps of:
- dry mixing powders of the composition ingredients in a planetary mixer for 10 minutes at a rotation speed of 60 rpm,
- granulating the mixture in the planetary mixer with water for 5 minutes at a rotation speed of 60 rpm,
- extruding the wetted mass by means of a Dome extruder (available from Fuji Paudal Co. , Tokyo, Japan; Dome die with perforation diameter 1 mm) operated at 45 rpm,
- spheronizing the extrudate by means of a spheronizer (Caleva, Dorset, United Kingdom) for 5 minutes at 1000 rpm followed by 10 minutes at 1250 rpm, and
- tray drying (35°C till constant weight) or fluid bed drying (using a dryer from Glatt, Bilzen, Germany; inlet temperature 50°C, outlet temperature 35°C).

### EXAMPLE 2 - Controlled release evaluation of the pellet composition

Drug release profiles were obtained by using the United States Pharmacopeia 23 paddle dissolution test. The following settings of the dissolution parameters were used :
Method : Paddle
Speed : 100 rpm
Medium : phosphate buffer pH 6.8
Sample time points : 5' 10' 15' 20' 30' 45' 60' 90' 120' 150'
Sample volume : 5 ml
Replacement medium : phosphate buffer pH 6.8
Detection : UV spectrophotometer
Wavelength : 353 nm
Results are expressed as the cumulative amount of drug released (in%) as a function of time.

### EXAMPLE 3 (comparative)

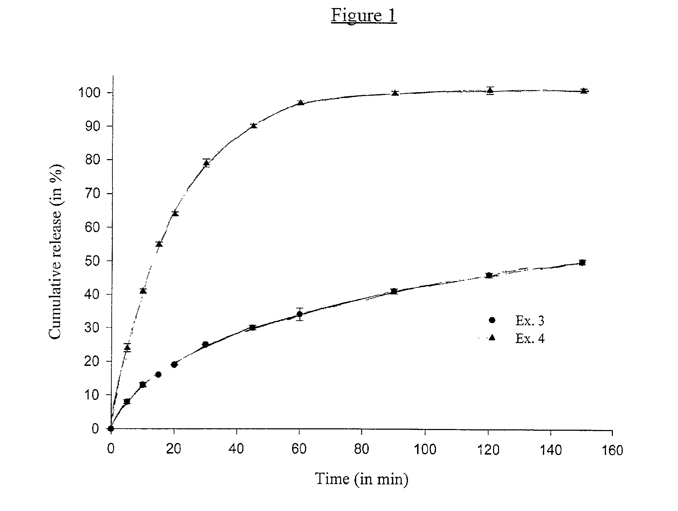

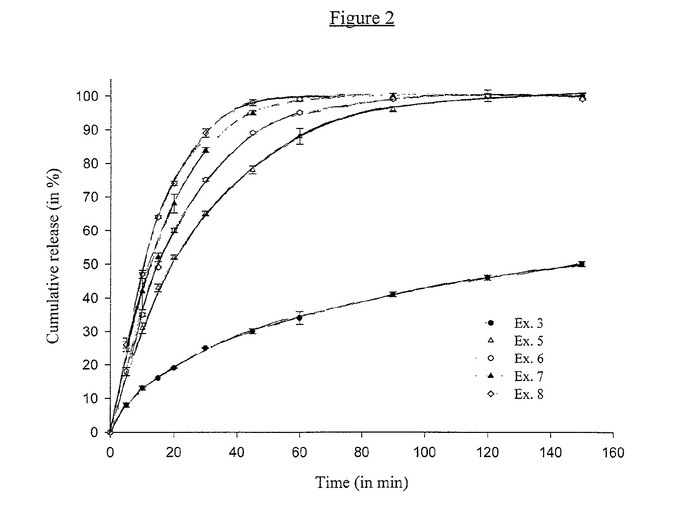

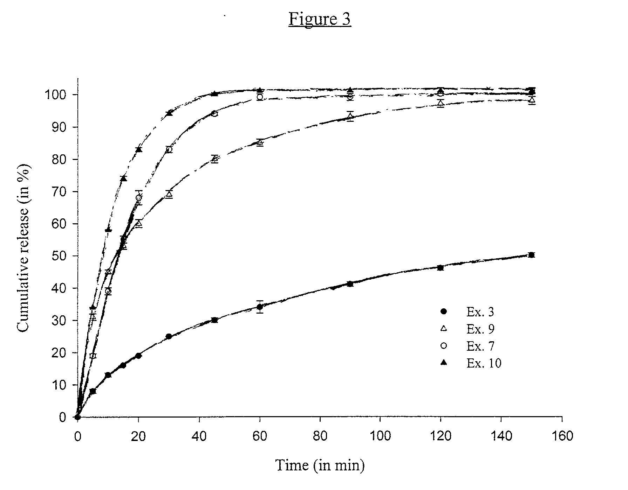

200 g of a pellet composition is produced, according to the method of example 1, from 5 g of piroxicam and 195 g of a microcrystalline cellulose available from FMC Corporation under the tradename Avicel ® PH 101. The composition is then evaluated according to the method of example 2. Results of evaluation are reported on figures 1 to 3 for comparison purposes with the pellet compositions of the present invention. The release of piroxicam from these pellets is rather slow, amounting to only 30% after 45 minutes.

### EXAMPLES 4 to 10

200 g of various pellet compositions are produced, according to the method of example 1, from 5 g of piroxicam and 195 g of a mixture of pellet components as indicated in the table below (all amounts expressed in grams).

**TABLE**

| Example | Avicel PH 101 | Swellable polymer containing component | Drug-dissolution enhancing agent |
|---|---|---|---|
| 4 | 0 | 195 (Avicel RC 581) | 0 |
| 5 | 97,5 | 97,5 (Avicel CL 611) | 0 |
| 6 | 65 | 130 (Avicel CL 611) | 0 |
| 7 | 48,75 | 146,25 (Avicel CL 611) | 0 |
| 8 | 0 | 195 (Avicel CL 611) | 0 |
| 9 | 155 | 0 | 40 (HPCD) |
| 10 | 38,75 | 116,25 (Avicel CL 611) | 40 (HPCD) |

Avicel RC 581 is a swellable polymer-containing component consisting of 86.2 to 91.7 % by weight of microcrystalline cellulose and 8.3 to 13.8% by weight of sodium carboxymethylcellulose.
Avicel CL 611 is a swellable polymer-containing component consisting of 81.2 to 88.7% by weight of a microcrystalline cellulose and 11.3 to 18.8% by weight of sodium carboxymethylcellulose.

### HPCD is an abbreviation for hydroxypropyl-β-cyclodextrin.

All compositions are then evaluated according to the method of example 2. Results of evaluation are reported on figures 1 to 3 respectively. They all demonstrate a release of at least 75% of piroxicam within 45 minutes in phosphate buffer pH 6.8.

## Claims

1. A controlled release pharmaceutical pellet composition comprising a non-steroidal anti-inflammatory drug, the said composition providing a release of at least 75% of the non-steroidal anti-inflammatory drug within 45 minutes in phosphate buffer pH 6.8, further comprising a microcrystalline cellulose and a swellable polymer in respective amounts such that the weight ratio of the swellable polymer to the microcrystalline cellulose is from 0.3 :100 to 30 :100.

2. A controlled release pharmaceutical pellet composition according to claim 1, wherein the swellable polymer is a carboxyalkylcellulose metal salt.

3. A controlled release pharmaceutical pellet composition according to claim 1 or claim 2, further comprising a drug dissolution enhancing agent.

4. A controlled release pharmaceutical pellet composition according to claim 3, wherein the drug dissolution enhancing agent is a cyclodextrin or a cyclodextrin derivative.

5. A controlled release pharmaceutical pellet composition according to any of claims 1 to 4, wherein the weight ratio of the drug dissolution enhancing agent to the microcrystalline cellulose is up to 50 :100.

6. A controlled release pharmaceutical pellet composition comprising a non-steroidal anti-inflammatory drug, the said composition providing a release of at least 75% of the non-steroidal anti-inflammatory drug within 45 minutes in phosphate buffer pH 6.8, further comprising a microcrystalline cellulose and a drug dissolution enhancing amount of a cyclodextrin derivative.

7. A controlled release pharmaceutical pellet composition according to claim 6, wherein the cyclodextrin derivative is hydroxypropyl-β-cyclodextrin.

8. A controlled release pharmaceutical pellet composition according to claim 6 or claim 7, wherein the cyclodextrin derivative and the microcrystalline cellulose are in respective amounts such that the weight ratio of the cyclodextrin derivative to the microcrystalline cellulose is at least 15 :100.

9. A controlled release pharmaceutical pellet composition according to any of claims 1 to 8, wherein the non-steroidal anti-inflammatory drug constitutes from 0.5 to 60% by weight of the composition.

10. A solid shaped article comprising a controlled release pharmaceutical pellet composition according to any of claims 1 to 9.

11. A solid shaped article according to claim 10, further comprising a coating.

12. A process for manufacturing a controlled release pharmaceutical pellet composition according to any of claims 1 to 9, comprising the steps of:
(a) granulating a mixture of a non-steroidal anti-inflammatory drug, a microcrystalline cellulose, a swellable polymer and/or a cyclodextrin derivative in the presence of a granulating fluid, and
(b) extruding the wet mass from step (a) by means of an extruder and then spheronizing the resulting extrudate by means of a spheronizer.

13. Use of a mixture comprising a microcrystalline cellulose and (a) a swellable polymer in respective amounts such that the weight ratio of the swellable polymer to the microcrystalline cellulose is from 0.3 :100 to 30 :100 and/or (b) a drug dissolution enhancing amount of a cyclodextrin derivative, for the manufacture of a medicament based on a non-steroidal anti-inflammatory drug and providing release of at least 75% of the said drug within 45 minutes in phosphate buffer pH 6.8.

14. A method for reducing side effects of a non-steroidal anti-inflammatory drug, comprising providing a dosage form including a controlled release pharmaceutical pellet composition according to any of claims 1 to 9 and administering said dosage form to a human or mammal patient orally.
